# EUROPEAN PATENT APPLICATION

(11) **EP 2 110 763 A1**
(43) Date of publication of application: **21.10.2009**
(21) Application number: 08007594.8
(22) Date of filing: 18.04.2008
(51) Int. Cl.: G06F 19/00

(54) **Health management feedback method using fitness equipments**

(71) Applicant: Strength Master Fitness Tech Co., Ltd., Wufeng Township Taichung (TW)
(72) Inventor: Su, Shen Yi, Taichung (TW)
(74) Representative: Strehl Schübel-Hopf & Partner

(57) **Abstract**

A health management feedback method by using fitness equipments comprises that a feedback unit 2 proposes at least one seductive health management feedback plan for feedback to user 1 and the health management feedback plan including the matching fitness equipment 3 and a feedback plan for setting the execution result 6 to realize seductive factors, wherein once user 1 using fitness equipment 3 being confirmed for self-use achieves the execution result 6 predefined by health management feedback system 5, feedback unit 2 is through feedback of the resource obtained by user 1 to realize seductive factors, and the predefined objective of the feedback plan proposed by feedback unit 2 is achieved through the execution result 6 done by user 1.

## Description

### BACKGROUND OF THE INVENTION

### (a) Field of the Invention

The invention is related to a health management feedback method using fitness equipments, more particularly to a health management feedback system to allow users to obtain feedback by using fitness equipments.

### (b) Description of the Prior Art:

As modern people are pursuing for body health, more and more people value the importance of exercises, and many people have the habit to use fitness equipments. However, it is often found that many people are unable to maintain using the fitness equipments persistently, i.e. users usually stop using the fitness equipments due to laziness or lack of time.

Regarding present market trend, there are many health management relevant institutions, wherein insurance companies are the most obvious. Usually, policyholders purchase an insurance policy from the insurance company for basic insurance protection, however, the insurance premium is usually dependent on health conditions of the policyholder, and the insurance company pays insurance compensation to the policyholder only when conditions of the policyholder conform to the contents of insurance policy. Therefore, it is only a passive insurance for a policyholder without more active benefits to the actual health of the policyholder.

Nevertheless, there are insurance companies who are cooperating with gym centers, wherein the policyholder visits the gym center cooperating with the insurance company for exercise, and the gym center stores presence records as evidence to obtain insurance compensation feedback. Further, there are insurance companies who establish interactive platforms on the internet, wherein the policyholder signs in to the individually independent account and enters the website platform to input his/her basic physical data during exercise period, while the physical data before and after the exercise are compared as the basis for insurance compensation feedback.

For both methods of the above described cooperation between insurance company and gym center or the internet website platform established by insurance company to interact with the policyholder, it is necessary to confirm the policyholder's identification to prevent cheating, i.e. the policyholder may find somebody else for substitution to visit the gym center for exercise, or fake his/her basic physical data in order to obtain insurance compensation feedback.

Hence, how to solve above said problems is the main emphasis by the invention.

### SUMMARY OF THE INVENTION

The above problems are solved by the method of the present invention as suggested in claim 1. The method facilitates data processing and exchange between fitness equipment, the health management feedback means and the feedback unit, and thus allows parties, such as insurances in the above example, to monitor and reward physical training of policyholders. Moreover, policyholders are placed in a position which allows them to profit from a healthy lifestyle also with respect to their financial resources.

The main purpose of the invention is to disclose a health management feedback method using fitness equipments, wherein through the use of the fitness equipment matched with feedback plan by user and further through the integration by health management feedback system , when the execution result by user conforms to the one predefined by feedback unit, the seductive (or award) factor is realized by the feedback unit to user, thereby to allow user not only to maintain a long term exercise habit, but also to obtain the feedback from the feedback unit, and the feedback plan objective of the feedback unit can also be achieved through the execution result by user 1.

To achieve above purpose, the invention includes that a feedback unit proposes at least one seductive health management feedback plan for feedback to user and the health feedback plan having an fitness equipment to match with the predefined objectives of the health management feedback plan is through internet to be online with a health feedback system for realizing results of the predefined seductive factors predefined by the feedback unit, wherein the execution result by the user using the fitness equipment being confirmed for self-use by the anti-cheating mechanism of health management feedback system during the feedback plan period is transmitted back to the feedback unit through the health management feedback system after the feedback plan is completed, once execution result conforms to the one predefined by the feedback unit, the seductive factors are then realized by the feedback unit 2 through feedback to the resource obtained by user.

### BRIEF DESCRIPTION OF THE DRAWINGS

Fig. 1 is a schematic view showing the feedback flow in the first embodiment of the invention.
Fig. 2 is a schematic view showing the feedback flow in the second embodiment of the invention.

### DETAIL DESCRIPTION OF THE PREFERRED EMBODIMENTS

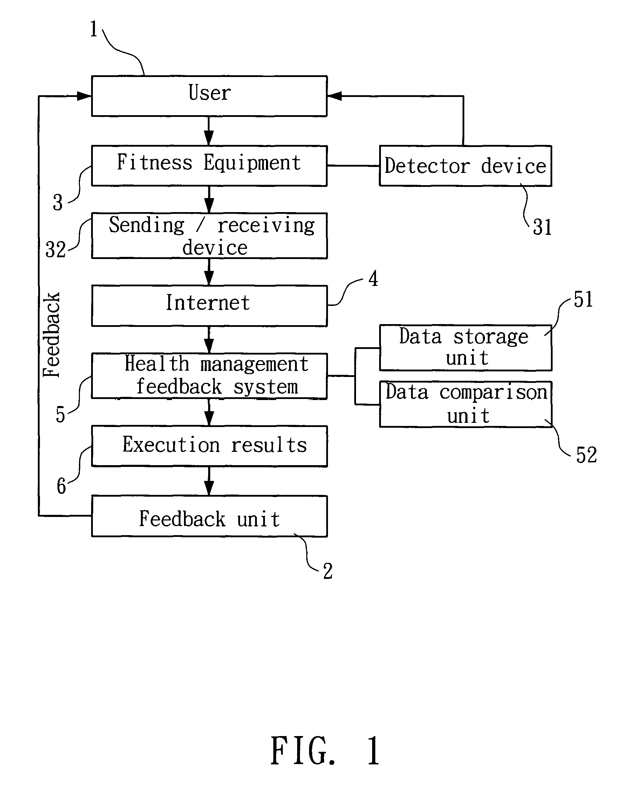

Referring to Fig. 1, wherein a selected embodiment of the invention is shown for description only, and is not intended to limit the scope of patent application.

A health management feedback method by using fitness equipments is disclosed in the embodiment which comprises that: a feedback unit 2 proposes at least one seductive health management feedback plan for feedback to user 1 and the health feedback plan having an fitness equipment 3 to match with the predefined objectives of the health management feedback plan is through internet 4 to be online with a health management feedback system 5 for realizing results of the predefined seductive factors predefined by the feedback unit 2, wherein, the execution result 6 by user 1 using fitness equipment 3 being confirmed for self-use by the anti-cheating mechanism of health management feedback system 5 during feedback plan period is transmitted back to feedback unit 2 through health management feedback system 5 after the feedback plan is completed, once execution result conforms to the one predefined by the feedback unit 2, the seductive factors are then realized by feedback unit 2 through feedback to the resource obtained by user 1.

In the embodiment, the fitness equipment 3 having a detector device 31 for detecting fitness data of user 1 during exercise is transmitted to health management feedback system 5 through a sending/receiving device 32 being online with internet 4, wherein health management system 5 can be managed by a specially assigned company, and it can also be monitored by a software or the internet unquestionably. The system 5 includes a data storage unit 51 for storing fitness data of user 1, and a data comparison unit 52 for comparing fitness data of user 1 and for matching with the anti-cheating mechanism, whereby fitness data of user 1 is stored and compared by the system 5 during feedback plan period to produce the execution result 6 after feedback plan period is over.

The fitness data of user 1 when using the fitness equipment for exercise is obtained and tracked by the health management feedback system 5 for comparison and analysis. wherein the fitness data includes not only the basic physical data such as weight, oxygen level in the blood, heat beats, blood pressure, etc. detected by the fitness equipment 3, but it also can be exercise parameters when the fitness equipment 3 is used by user 1 (such as running speed detected by a speed detector, running time detected by a timer, or running mileage detected by an odometer, etc. when a running machine is used by user 1).

In case that the fitness data of user 1 in exercise is pulse, wherein each time the pulse of user 1 using the fitness equipment 3 being detected by detector device 31 to draw the pulse wave and being stored in data storage unit 51 is accessed by the anti-cheating mechanism of health management feedback system 5 for comparing with the pulse wave of user 1 through data comparison system 52, thereby to confirm the self-use of user 1 with fitness equipment 3.

Storage unit 51 setting value cannot be revised after initial setting is done. The sending/receiving device 32 is a computer system which is through a storage interface (such as USB disk or SD memory card, etc) to access fitness data of user 1 from fitness equipment 3 and save it back to the sending/receiving device 32 for transmission to health management feedback system 5 through internet 4 for analysis and comparison. Further, sending/receiving device 32 can be directly mounted in fitness equipment 3 and online with internet 4 to directly transmit fitness data of user 1 to health management feedback system 5 for analysis and comparison.

For instance, if feedback unit 2 is an insurance company for communication with health management feedback system 5 through internet 4, the feedback plan is an insurance policy which is either a new one to be joined by user 1, or an existing one for obtaining feedback, the resource obtained by insurance company is the insurance premium paid by user 1, wherein the seductive factor proposed by insurance company is to provide a premium discount to user 1 when the execution result 6 defined by the feedback plan is reached by using fitness equipment 3. If the feedback plan period is one year and fitness equipment 3 is an electrical running machine, pre-defined result of feedback unit 2 is that user 1 is required to maintain exercise in five days per week and minimum one hour per day within the one year period, wherein pulse of user 1 in exercise using fitness equipment 3 is detected by a detector device 31 installed thereon to obtain a pulse wave shape, whereby physical data of user 1 is further through sending/receiving device 32 to be directly or indirectly transmitted back to health management feedback system 5 through internet 4 for access by data storage unit 51 of system 5 to compare with the pulse wave shape of user 1 for self-use in the exercise. After the one-year feedback period is over, the execution result 6 of five days per week and minimum one hour per day exercise is transmitted back to feedback unit 2 through health management feedback system 5, and if execution result 6 conform to the one predefined by feedback unit 2, the feedback unit 2 then provide premium discount feedback to user 1 due to realization of the seductive factor. Regarding fitness equipment 3, besides the electrical running machine used for exercise by user 1 in the embodiment, the fitness equipment can also be a detector for detecting the basic physical data of user 1 such as a pedometer, a blood pressure monitor, or a blood sugar machine, etc., wherein basic physical data of user 1 can be transmitted by a transmission interface (such as USB) to health management feedback system 5.

Therefore, through the use of fitness equipment 3 matched with feedback plan by user 1 and further through the integration by health management feedback system 5, when execution result 6 by user 1 conforms to the one predefined by feedback unit 2, the seductive factor is realized by feedback unit 2 to user 1, thereby to allow user 1 not only to maintain a long term exercise habit, but also to obtain the feedback from the feedback unit, and the feedback plan objective of the feedback unit can also be achieved through the execution result 6 by user 1. Of course, as pulse of user 1 is confirmed by anti-cheating mechanism of the invention, the self-use of fitness equipment 3 by user 1 can be ensured to prevent any possibilities of faking identity of user 1 to obtain feedback.

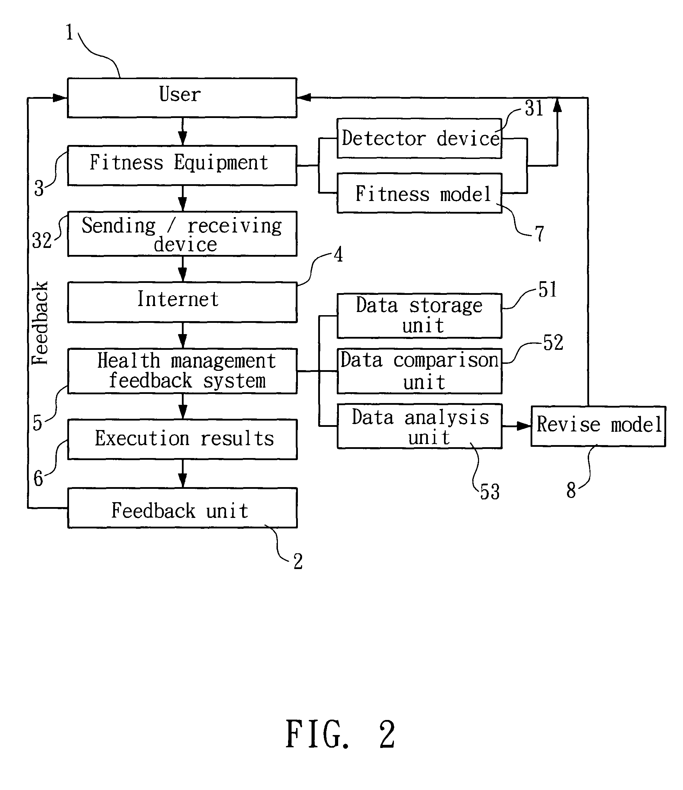

Of course, there exist many examples of the invention with only detail variations. The second embodiment of the invention is shown in Fig. 2, wherein health management feedback system 5 is further installed with a data analysis unit 53 for analyzing the fitness data of user 1 which is obtained by health management feedback system 5 to define a fitness model 7 on the fitness equipment 3, so that physical data of user 1 under fitness model 7 is routinely tracked by health management feedback system 5 is compared and analyzed by health management feedback system 5 to produce execution result 6 after the feedback plan period is over.

The fitness model 7 is the setting model for fitness equipment use by user 1 defined at initial feedback period, such as the applicable parameters setting for the fitness equipment according to user' s health condition in the initial use.

During the exercise period of user 1 under fitness model 7, fitness data of user 1 in exercise is analyzed and compared by the health management feedback system 5 and is further routinely provided with a feedback by modification model 8, wherein fitness data of user 1 in exercise is analyzed and compared with the predefined healthy physical data (analysis of physical data changes such as weight, oxygen level in blood, heart beat, blood pressure, etc.) through data analysis unit 53 and data comparison unit 52 to produce the routine modification model 8 thus allowing user 1 to modify the initial fitness mode 7 of fitness equipment 3 for advance use. Such as that fitness data of user 1 after exercise for three months shall be better than the initial one, therefore, the original fitness model 7 is revised to the more difficult modification model 8. The modification model 8 is transmitted back to fitness equipment 3 through internet 4 to modify the initial fitness model 7.

As can be seen in the embodiment, besides that long term exercise habits of user 1 in first embodiment can be achieved, it can also provide challenging or different degree of pleasure of user 1 in using fitness equipment 3 through the revision of fitness model 7 during feedback period.

Of course, feedback unit 2 referred by the invention is not limited to insurance company only, such as that it can be an fitness equipment supplier company for communication with health management feedback system 5 through internet 4, wherein the resource obtained by fitness equipment supplier company is the price for user 1 to buy fitness equipment 3, while the seductive factor of health management feedback plan proposed by fitness equipment supplier company is the feedback money given to user 1 when the execution result 6 defined by feedback plan is achieved by using fitness equipment 3.

## Claims

1. A health management feedback method in a health management system, the system comprising
fitness equipment (3),
a feedback unit (2), and
health management feedback means (5) connected to the fitness equipment and the feedback unit via a computer network (4),
wherein the method comprises:
setting up and storing a health management feedback plan for a user (1) in the feedback unit (2), and transmitting it to the health management feedback means (5);
transmitting fitness data for monitoring the use of the fitness equipment (3) by the user (1) during a period specified by the health management plan from the fitness equipment (3) to the health management feedback means (5), and
processing the fitness data in the health management feedback means (5) in order to ensure that the user (1) does not contravene the specifications of the health management feedback plan in using the fitness equipment (3);
transmitting, once the feedback plan and the matching of the fitness data are completed, execution result data (6) to the feedback unit (2)
processing the execution result data (6) in the feedback unit (2) in order to match them with the predefined objects in the health management feedback plan and, if the match has been successful, providing the user (1) with feedback on an award factor defined by the health management feedback plan and concerning a resource of the user (1).

2. The method of claim 1,
wherein the fitness equipment (3) comprises a detector device (31) to measure the physical data of the user (1) for transmission to the health management feedback means (5) through a transmitter/receiver device (32) via the computer network (4),
wherein the health management feedback means (5) further includes a storage unit (51) for saving fitness data of the user (1) and a data comparison unit (52) provided with an anti-cheating mechanism for processing and comparing the fitness data of the user (1), such that the fitness data of the user (1) are stored and compared during the feedback plan period in order to produce execution result data (6) after the plan period is over.

3. The method of claim 1 or 2, wherein the fitness data are basic physical data of the user (1) detected while using the fitness equipment (3).

4. The method of claim 3, wherein the basic physical data of the user (1) are pulse data and the processing of the fitness data includes the analysis of pulse waves based on the pulse data and pre-stored pulse waves.

5. The method of claim 2, wherein the storage unit settings cannot be revised after initialisation.

6. The method of claim 1, wherein the fitness data of the user (1) are obtained by the health management feedback means (5), and physical data of the user (1) in exercise are tracked, compared and analysed by the health management feedback means (5) to produce execution result data (6) when the feedback plan period is over.

7. The method of any of the preceding claims, wherein the fitness data of the user (1) obtained by the health management feedback means (5) define a fitness model (7) for the exercise of the user (1) on the fitness equipment (3), and physical data of the user (1) in exercise under fitness model (7) are routinely tracked, compared and analysed by the health management feedback means (5) to produce the execution result data (6) when the feedback plan period is over.

8. The method of claim 7, wherein a first version of the fitness model (7) is initially set, while the fitness data of the user (1) in exercise are analysed and compared with predefined healthy physical data by the health management feedback means (5) to routinely produce a short time modification model (8) during the feedback plan period in order to modify the initial fitness model (7) of the fitness equipment (3) thereby allowing user (1) to exercise under a modified fitness model.

9. The method of claim 8, wherein the modification model (8) is transmitted by health management feedback means (5) back to the fitness equipment (3) through the computer network (4) in order to modify the initial fitness model (7).

10. The method of any of the preceding claims, wherein the fitness equipment (3) further comprises a data analysis unit (53) for analysing the fitness data of the user (1), whereby fitness data of user (1) are stored, compared and analysed during feedback plan period to produce execution result data (6), when the feedback plan period is over.

11. The method of claim 10, when dependent on claim 7, wherein the fitness data of the user (1) are analysed and compared with predefined healthy physical data by the data analysis unit (53) and the data comparison unit (52) to produce the routine modification model (8).

12. The method of any of the preceding claims, wherein the feedback unit (2) is associated with an insurance company and the feedback plan is defined on the basis of an insurance policy which is either a new one to be acquired by the user (1) or an existing one, while the resource of the user (1) is an insurance premium paid by him and the award factor proposed by the insurance company is the provision of a premium discount to the user (1).

13. The method of any of the preceding claims, wherein the computer network (4) is the internet.

## Amended claims

### Amended claims in accordance with Rule 137(2) EPC.

**1.** A health management feedback method in a health management system, in which health management feedback means (5) are connected to a fitness equipment (3) and a feedback unit (2) via a computer network (4), wherein the method comprises:
setting up and storing a health management feedback plan for a user (1) in the feedback unit (2), and transmitting it to the health management feedback means (5);
transmitting fitness data monitoring the use of the fitness equipment (3) by the user (1) during a period specified by the health management plan from the fitness equipment (3) to the health management feedback means (5), and
processing the fitness data in the health management feedback means (5) in order to ensure that the user (1) does not contravene the specifications of the health management feedback plan in using the fitness equipment (3);
transmitting, once the feedback plan and the matching of the fitness data are completed, execution result data (6) to the feedback unit (2);
processing the execution result data (6) in the feedback unit (2) in order to match them with predefined objects in the health management feedback plan and, if the match has been successful, providing the user (1) with feedback on an award factor defined by the health management feedback plan and concerning a resource of the user (1).

**2.** The method of claim 1,
wherein the fitness equipment (3) comprises a detector device (31) to measure the physical data of the user (1) for transmission to the health management feedback means (5) through a transmitter/receiver device (32) via the computer network (4),
wherein the health management feedback means (5) further includes a storage unit (51) for saving fitness data of the user (1) and a data comparison unit (52) provided with an anti-cheating mechanism for processing and comparing the fitness data of the user (1), such that the fitness data of the user (1) are stored and compared during the feedback plan period in order to produce execution result data (6) after the plan period is over.

**3.** The method of claim 1 or 2, wherein the fitness data are basic physical data of the user (1) detected while using the fitness equipment (3).

**4.** The method of claim 3, wherein the basic physical data of the user (1) are pulse data and the processing of the fitness data includes the analysis of pulse waves based on the pulse data and pre-stored pulse waves.

**5.** The method of claim 2, wherein the storage unit settings cannot be revised after initialization.

**6.** The method of claim 1, wherein the fitness data of the user (1) are obtained by the health management feedback means (5), and physical data of the user (1) in exercise are tracked, compared and analyzed by the health management feedback means (5) to produce execution result data (6) when the feedback plan period is over.

**7.** The method of any of the preceding claims, wherein the fitness data of the user
(1) obtained by the health management feedback means (5) define a fitness mode (7) for the exercise of the user (1) on the fitness equipment (3), and physical data of the user (1) in exercise under fitness mode (7) are routinely tracked, compared and analyzed by the health management feedback means (5) to produce the execution result data (6) when the feedback plan period is over.

**8.** The method of claim 7, wherein a first version of the fitness mode (7) is initially set, while the fitness data of the user (1) in exercise are analyzed and compared with predefined healthy physical data by the health management feedback means (5) to routinely produce a short time modification mode (8) during the feedback plan period in order to modify the initial fitness mode (7) of the fitness equipment (3) thereby allowing user (1) to exercise under a modified fitness mode.

**9.** The method of claim 8, wherein the modification mode (8) is transmitted by health management feedback means (5) back to the fitness equipment (3) through the computer network (4) in order to modify the initial fitness mode (7).

**10.** The method of any of the preceding claims, wherein the fitness equipment (3) further comprises a data analysis unit (53) for analyzing the fitness data of the user (1), whereby fitness data of user (1) are stored, compared and analyzed during feedback plan period to produce execution result data (6), when the feedback plan period is over.

**11.** The method of claim 10, when dependent on claim 7, wherein the fitness data of the user (1) are analyzed and compared with predefined healthy physical data by the data analysis unit (53) and the data comparison unit (52) to produce the routine modification mode (8).

**12.** The method of any of the preceding claims, wherein the feedback unit (2) is associated with an insurance company and the feedback plan is defined on the basis of an insurance policy which is either a new one to be acquired by the user (1) or an existing one, while the resource of the user (1) is an insurance premium paid by him and the award factor proposed by the insurance company is the provision of a premium discount to the user (1).

**13.** The method of any of the preceding claims, wherein the computer network (4) is the internet.
